Europäisches Patentamt

European Patent Office

Office européen des brevets

(11) Publication number: **0 161 866 B1**

# EUROPEAN PATENT SPECIFICATION

(45) Date of publication of patent specification: **01.07.92**  (51) Int. Cl.⁵: **C12N 15/57**

(21) Application number: **85303085.6**

(22) Date of filing: **01.05.85**

The file contains technical information submitted after the application was filed and not included in this specification

(54) **Human renin genomic DNA.**

(30) Priority: **03.05.84 US 606723**

(43) Date of publication of application:
**21.11.85 Bulletin 85/47**

(45) Publication of the grant of the patent:
**01.07.92 Bulletin 92/27**

(84) Designated Contracting States:
**BE CH DE FR GB IT LI NL SE**

(56) References cited:
**EP-A- 0 135 347**
**EP-A- 0 196 921**

**PROC. NATL. ACAD. SCI. USA, vol. 80, December 1983, pages 7405-7409; T. IMAI et al.: "Cloning and sequence analysis of cDNA for human renin precursor"**

**IDEM**

**DRUG DEVELOPMENT RESEARCH, vol. 1, 1981, pages 435-454, Alan R. Liss, Inc.; W.L. MILLER et al.: "Synthesis of biologically active proteins by recombinant DNA technology"**

**PROC. NATL. ACAD. SCI. USA, vol. 81, October 1984, pages 5999-6003; H. MIYAZAKI et al.: "Structure of the human renin gene"**

**IDEM**

**CHEMICAL ABSTRACTS, vol. 99, no. 15, 10th October 1983, page 182, abstract no. 117073m, Columbus, Ohio, US; C. GORMAN et al.: "High efficiency DNA-mediated transformation of primate cells", & SCIENCE (Washington, D.C., 1883-) 1983, 221(4610), 551-3**

(73) Proprietor: **Murakami, Kazuo**
**116-103 Namiki-2-chome Sakura-mura**
**Niihari-gun Ibaraki Prefecture(JP)**

(72) Inventor: **Murakami, Kazuo**
**116-103 Namiki-2-chome Sakura-mura**
**Niihari-gun Ibaraki Prefecture(JP)**

(74) Representative: **Perry, Robert Edward et al**
**GILL JENNINGS & EVERY 53-64 Chancery Lane**
**London WC2A 1HN(GB)**

Rank Xerox (UK) Business Services

## Description

Renin is an aspartyl proteinase synthesized mainly in the juxta-glomerular cells of the kidney, which are located in the afferent arterioles immediately proximal to the glomeruli (Oparil, S. and Haber, E., N. Engl. J. Med. 291, 389-401, 1974). When the blood flow through the kidney becomes inadequate, renin is secreted into the circulation where it acts on a plasma protein to produce the decapeptide angiotensin I. Under the influence of the converting enzyme, the angiotensin I derived from the larger parent protein angiotensinogen, in turn, becomes angiotensin II which has potent vasoconstrictor and aldosterone secretion activities. Renin is, therefore, the key enzyme of the renin-angiotensin-aldosterone cascade aimed at increasing blood pressure and extracellular fluid volume (Reid, I.A., et al., Ann. Rev. Physiol. 40, 377-410, 1978).

Imai, T., et al., Proc. Natl. Acad. Sci. USA 80, 7405-7409 (1983) have reported the isolation and sequence analysis of a human renin cDNA clone. From the cDNA sequence, the human renin precursor, preprorenin, was shown to be composed of 406 amino acids. Using human renin cDNA to isolate the corresponding gene sequence from a human genomic library, analysis of the organization and structure of the renin gene revealed that it exists in a single copy, spans roughly 11.7 kb, and consists of 10 exons.

The present invention provides human renin genomic DNA in a recombinant DNA sequence and an animal host cell transformed thereby.

In the drawings;

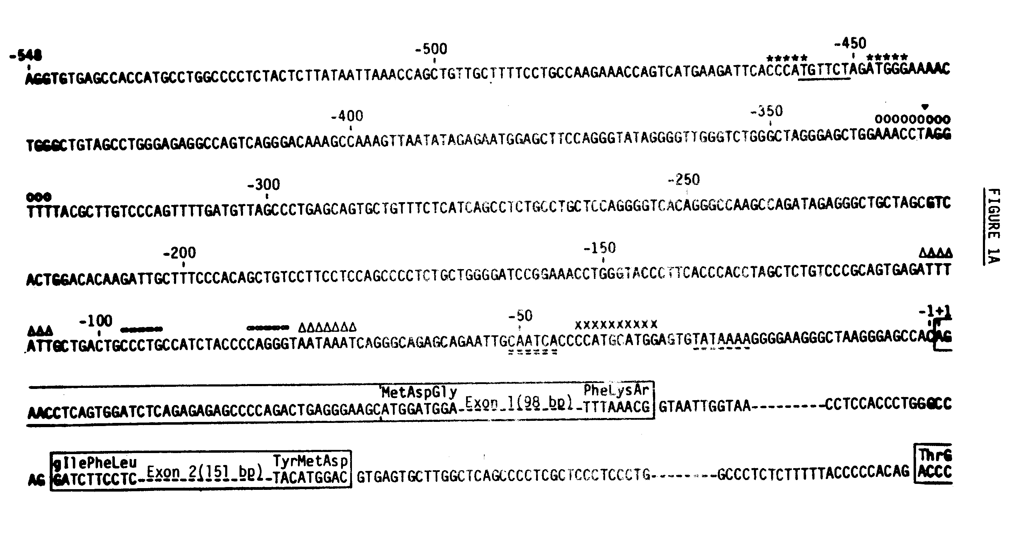

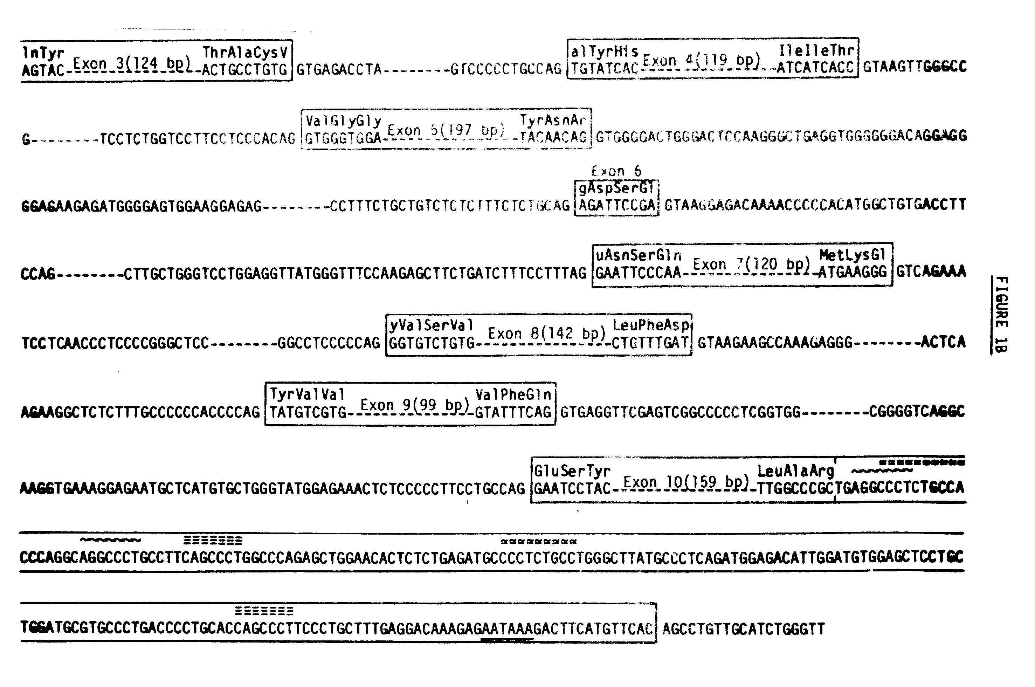

Figs. 1A and 1B. DNA sequence of the human renin gene. Position +1 corresponds to the transcription initiation site. Exons are boxed. The CAAT, TATA, and AATAAA sequences are underlined. The hexanucleotide TGTTCT, which is considered to be the glucocorticoid-receptor-binding sequence, is indicated by bold line. Palindromic sequences in the 5'-flanking region are marked by dots, star, closed triangles, and open squares. Repeated sequences in the 3'-noncoding region are indicated by various lines.

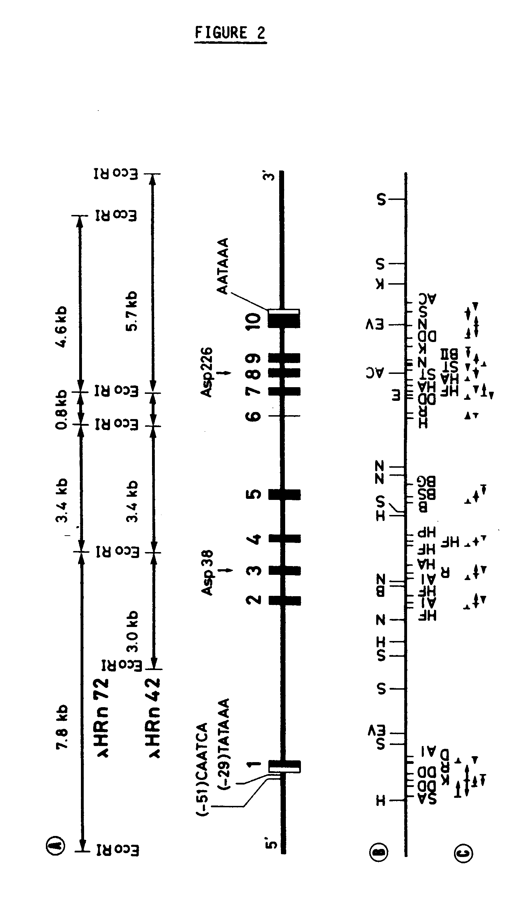

Fig. 2. Restriction map and strategy for sequencing the human renin gene. A, location of EcoRI sites in recombinant phage clones. B, detailed restriction map of the renin gene and its flanking regions. The restriction sites utilized for sequencing are indicated by downward lines. C, regions of the DNA which were sequenced; the horizontal arrows indicate the direction and length of each sequence analysis. The structure of the renin gene is schematically illustrated with solid (coding regions) and open bars (untranslated regions) and solid lines (introns and flanking regions). All sites of the following restriction enzymes are shown: B, BamHI; EV, EcoRV; H, HindIII; K, KpnI; S, SacI. Restriction sites used for sequencing are: AC, AccI; AI, AvaI; BII, BanII; BG, BglI; BS, BstEII; D, DraI; DD, DdeI; E, EcoRI; HA, HaeIII; HF, HinfI; HP, HpaII; N, NcoI; R, RsaI; SA, Sau3AI; ST, StuI (these do not cover all the sites present in the gene).

## SUMMARY OF INVENTION

This invention is a sequence of recombinant DNA base pairs encoding human renin comprising the sequence of DNA base pairs set forth in Figures 1A and 1B hereof and functional equivalents thereof and an animal cell transformed with said replicable sequence of recombinant DNA.

## DETAILED DESCRIPTION OF INVENTION

The human renin gene was isolated from a Charon 4A-human genomic library and characterized. The gene spans about 11.7 kb and consists of 10 exons and 9 introns which map at variable surface loops of the enzyme. The complete coding regions, the 5'- and 3'- flanking regions, and the exon-intron boundaries were sequenced. The active site aspartyl residues Asp 38 and Asp 226 are encoded by the third and eighth exons, respectively. The extra three amino acids (Asp 165 - Ser 166 - Glu 167) which are not present in mouse renin are coded for by the separate sixth exon, an exon as small as 9 nucleotides. The putative TATA and CAAT sequences, which may play a role in the initiation of gene transcription, are found in the vicinity of -29 and -51 nucleotides of the CAP site. Further upstream, at nucleotides -456 to -451, is located the hexanucleotide TGTCT which has recently been suggested as a binding site for the glucocorticoid receptor. In the 3'-flanking region there is the conserved hexanucleotide sequence, AATAAA, thought to be necessary for polyadenylation. Blot-hybridization analyses of the isolated gene clone and the total cellular DNA after digestion with restriction enzymes revealed that human renin is encoded by a single gene.

The structure of the genomic human renin gene is set forth in Figures 1A and 1B and is described in detail hereinbelow. The following plasmids containing fragments of the genomic human renin gene were constructed as described in detail hereinbelow. Refer to Figures 1A, 1B and 2 for an identification of the gene region contained in each plasmid:

| | |
|---|---|
| pHRg07: | the 0.7 kb internal EcoRI fragment |
| pHRg34: | the 3.4 kb internal EcoRI fragment |
| pHRg78: | the 5' end of the gene as a 7.8 kb EcoRI fragment |

pHRg88:    the 3' end of the gene as an 8.8 kb
           Hind III fragment

pHRg07 is available from Escherichia coli HB101, NRRL-B-15765.

pHRg34 is available from Escherichia coli HB101, NRRL-B-15766.

pHRg78 is available from Escherichia coli HB101, NRRL-B-15767.

PHRg88 is available from Escherichia coli HB101, NRRL-B-15768.

Biologically pure cultures of each of the above strains were deposited on 23.04.84 at the permanent collection of the Northern Regional Research Center Fermentation Laboratory (NRRL), U.S. Department of Agriculture in Peoria, Illinois, USA.

The renin gene fragments can be extracted from the above-identified plasmids by digesting the plasmid DNA from a culture(s) of NRRL B-15765, B-15766, B-15767, or B-15768 with appropriate endonuclease restriction enzymes, most suitably enzymes which will cleave at the sites of the gene depicted in Figure 2. The restriction endonucleases are commercially available and their use is well known in the art and usually is provided by the commercial supplier of the enzyme. The excised gene setment(s) can be ligated to various cloning vehicles or vectors for use in transforming a host cell. The vector may be a replicating vector, i.e., a vector which replicates within the host cell, or an integrating vector, i.e., a vector which serves to transport the exogenous gene material into the host cell and integrate said gene material with the DNA of the host cell. The vector contains heterologous gene sequences to initiate transcription and translation of the renin gene which are compatible with the host cell to be transformed. There are several possible ways to use genomic clones of the human renin gene to obtain human renin expressed from animal cells. For example, the intact genomic clone, with its own promoter and polyadenylation signal can be inserted into a cell by any one of the following types of vector systems: co-transfection of host cells with the renin gene and a marker for which there is a selection, e.g., the herpes simplex virus thymidine kinase gene as generally described in Cell, 16, 777-785 (1979); insertion of the renin gene into a vector which integrates into cellular DNA and confers on the cell some selectable phenotype, e.g., pSV2neo as generally described in J. Mo. Appl. Genet. 1, 327-341 (1982); or insertion of the renin gene into a replicating vector such as bovine papilloma virus as generally described in Proc. Natl. Acad. Sci. USA 80, 4040-4044 (1983). Additionally, to obtain higher levels of renin expression the natural renin promoter can be replaced with a known strong promoter such as the SV40 early promoter (see, e.g., Science 222, 524-527 (1983)) or one of the many other promoters that are known to work efficiently in animal cells. Similarly, the polyadenylation site of the renin gene can be replaced with one from a highly expressed gene such as the mouse alpha globin gene (e.g., Gene, 24, 2281-287 (1983)) to enhance renin expression. Any modified transcription units could be introduced into cells by the three types of methods described above. In essence one can use any sequence of DNA base pairs encoding genomic human renin. By base pairs is meant the text book definition of adenine (A) and thymine (T) and cytosine (C) and guanine (G) as occurs in DNA sequences.

There are several well-known methods of introducing DNA into animal cells, and these may be used for any of the three types of vector systems described above. These include: calcium phosphate precipitation of the DNA and transfection with the calcium phosphate precipitate, fusion of the recipient cells with bacterial protoplasts containing the DNA, treatment of the recipient cells with liposomes containing the DNA, and microinjection of the DNA directly into the cells. The transformed cells are grown up by means well known in the art, e.g., see Biochemical Methods in Cell Culture and Virology by R.J. Kuchler, Dowden, Hutchinson and Ross, Inc., 1977. The following example further illustrates the invention.

The present invention provides a source of human genomic renin which can be used in diagnosing hypertensive patients by assaying the patients sera with antibodies to the human renin by various known immunoassay techniques. Also, the renin expressed from the human genomic renin gene expressed in accordance with the present invention can be used to design inhibitors of renin which would be useful in treating hypertensive patients.

EXAMPLES

Example 1:

Materials. All restriction enzymes were obtained from either New England BioLabs or Takara Shuzo (Kyoto, Japan). Escherichia coil alkaline phosphatase and T4 DNA ligase were from Takara Shuzo. [γ-$^{32}$P]ATP (>5,000 Ci/mmol) and [α-$^{32}$P]-dCTP ≈3,000 Ci/mmol) were from Amersham.

All cloning experiments were performed in accordance with the guidelines for research involving recombinant DNA molecules issued by the Ministry of Education, Science and Culture of Japan.

Screening. A human genomic library, prepared from partial Alu I and Hae III digestion and ligated into the EcoRI arms of the λ vector Charon 4A, was provided by and is described by Dr. Maniatis (Lawn, R.M., et al., Cell 15, 1157-1174, 1978). A portion of the amplified library plated on E. coli LE 392 was screened, by the method of Benton and Davis (Benton, W.D. and Davis, R.W., Science 196, 180-182, 1977), for sequences that hybridize with cDNA probes (Pst I - Hpa II and Hpa II - Rsa I fragments of pHRn 321 (Imai, T., et al., Proc. Natl. Acad. Sci. USA 80, 7405-7409, 1983)) labeled with $^{32}$P by nick-translation to a specific activity of 2 x 10$^8$ cpm/$\mu$g. Hybridization was conducted at 65°C for 12 to 24 hours and the filters were sequentially washed several times in (i) 2 x SSC (1 x SSC is 0.15 M NaCl, 0.015 M Na-citrate) containing 0.1% SDS at room temperature; (ii) 2 x SSC, 0.1% SDS at 65°C; (iii) 0.1 x SSC, 0.1% at 65°C; and (iv) 2 x SSC at room temperature; and exposed to Fuji X-ray film RX-50 with a DuPont Cronex Lightning Plus screen. 400,000 recombinant phages were screened and 7 positive clones were identified and rescreened at low plaque density to achieve high purity. The preparation of phage DNA were carried out as generally described by Maiatis, T., et al., Cell 15, 678-701 (1978).

Subcloning. DNA fragments from the positive phage clones were digested with EcoRI. Preliminary restriction enzyme analysis of these clones indicated that they covered similar regions of about 12 kb of the human genome. Two clones, designated $_\lambda$HRn 42 and $_\lambda$HRn 72, respectively, were further analyzed and shown to have three EcoRI sites (Figure 2). The orientation of the gene was determined by using the 5' and 3' specific cDNA probes generated by digesting the human renin cDNA at a unique Hpa II site. Four EcoRI fragments (the 5'-most 7.8 - kb, the 3'-most 5.7 - kb, and the overlapping 3.4 - kb and 0.8 - kb fragments shown at A in Figure 2) which together comprise 17.7 kb of the human genome were isolated by electrophoresis through 0.7% agarose gels.

The purified DNA fragments were subcloned into the EcoRI site of plasmid pBR 322. Transformations of E. coli HB101 were performed as described by Morrison (Morrison, D.A., Methods Enzymol. 68, 326-331, 1979) to give E. coli HB101 (pHRg07); E. coli HB101 (pHRg34); E. coli HB101 (pHRg78); and E. coli HB101 (pHRg88).

Mapping and Sequencing. A partial restriction map of the human renin gene was constructed and is depicted in Figure 2. DNA samples were digested with various restriction enzymes under conditions specified by the supplier and separated by electrophoresis on 0.7% and 2.5% agarose or 5% polyacrylamide gels. Ethidium bromide-stained gels were photographed under UV light and DNA was transferred to nitrocellulose filters in 6 x SSC as described by Southern (Southern, E.M., J. Mol. Biol. 98, 503-517).

The restriction fragments containing an exonic sequence and its surrounding regions were identified by blot hybridization at 65°C for 12 hours with a nick-translated $^{32}$P-cDNA probe as described above. Filters were autoradiographed at -70°C with intensifier screens. The fragments were isolated from polyacrylamide gels and subjected to nucleotide sequence analysis to determine the exon-intron junctions by the procedure of (Maxam and Gilbert, Methods Enzymol. 65, 499-560 (1980)). The sequence strategy used is shown in Figure 2. As shown in Figures 1A, 1B and 2, human renin gene consists of 10 exons and 9 introns. Central parts of the introns were not sequenced. The exon sequences are identical with those of the corresponding region of the cDNA (Imai, T., et al., Proc. Natl. Acad. Sci. USA 80, 7405-7409, 1983). In accordance with the general GT-AG rule, all of the introns begin with the dinucleotide GT and end with AG. More extensive consensus sequences have been proposed for the RNA splice sites by analyzing a great number of eukaryotic genes that are transcribed by RNA polymerase II (Breathnach, R. and Chambon, P., Ann. Rev. Biochem. 50, 349-383, 1981, and Mount, S.M., Nucleic Acids Res. 10, 459-472). The donor $\binom{C}{A}$ AG/GT$^A_G$ ATGT) and the acceptor (($^T_C$)$_{\sqcup\sqcup}$N$^C_T$ AG/G) consensus sequences are closely followed by the intron-exon junctions of the renin gene (Figures 1A and 1B).

Comparison between the gene structure and the tertiary structure of human renin (Umeyama, H., et al., Hypertension, 1984, in press) revealed that all the intron-exon boundaries follow the rule, proposed by Craik, et al. (Craik, (C.S., Nature 299, 180-182, 1982), that splice junctions tend to map at protein surfaces.

Soubrier, et al (Soubrier, F., et al., Nucleic Acids Res. 11, 7181-7190, 1983) have shown that the renin gene exists in a single copy in the human genome. To confirm this and to establish the absence of major sequence rearrangement of the renin gene during cloning and propagation of the $_\lambda$HRn 72 DNA, restricted genomic DNA was analyzed by blot hybridization. When the placental cellular DNA was digested with Sac I or HindIII the fragments detected by the $^{32}$pP-labeled cDNA probe were identical with those seen in the cloned $_\lambda$HRn 72 DNA except the fragments containing the left or right arm of $_\lambda$ phage DNA. The sizes of these hybridizing fragments correspond exactly to those expected from the partial restriction map shown in Figure 2. The cloned gene sequence ($_\lambda$HRn 72) represents the original structure of the human renin gene which is present as a single copy in the chromosome.

Flanking Regions. The 3'- and 5'-flanking regions, with a special emphasis on the 5' portion, were also sequenced since these regions may be important in the regulation of gene expression. The transcription initiation site or the CAP site was tentatively assigned to the residue A labeled +1 based on the fact that an "A", which is preceded by a "C" in most cases, is the preferred CAP site and assuming that our cDNA covers nearly full length of its template, namely, human renin mRNA. This assignment is supported by the relative position of the putative regulatory sequences such as Goldberg-Hogness box (TATA) found upstream of the 5' terminus. Inspection of the region upstream from the start site revealed the presence of the promotor sequence, TATAAA, between positions -29 and -24. At positions -51 to -46 there is the sequence CAATCA which is homologous to the consensus "CAAT" box. In the region immediately upstream from the TATA box several palindromic sequences are observed as indicated in Figures 1A and 1B. Further upstream is located a hexanucleotide sequence, TGTTCT, which has been shown to be a preferential binding site for the glucocorticoid receptor using cloned DNA fragments of mouse mammary tumor virus (Scheidereit, C., Nature 304, 749-752, 1983).

Example 2:

The plasmid pHRg88 is digested with EcoRI, the 5.7 kb EcoRI fragment isolated, and that fragment cloned into the EcoRI site of pSV2neo. The resulting plasmid is partially digested with EcoRI, full-length linear molecules isolated, and the 0.7 kb EcoRI fragment from pHRg07 cloned into that. Using restriction enzymes cleavage sites shown in Figure 2, the resulting plasmids are screened for ones containing two EcoRI fragments oriented the same as indicated in Figure 2 to exist in the human renin gene. This process is repeated on the resulting plasmid to insert the 3.4 kb EcoRI fragment from pHRg34; and then the same thing is done to insert the 7.8 kb EcoRI fragment from pHRg78. The resulting plasmid carries the entire human renin gene.

The resulting plasmid carrying the intact renin gene can be introduced into Hela cells by calcium phosphate precipitation. 10 $\mu$g of plasmid DNA in 0125 ml 10 mM TrisHCl, pH 7.4, 1mM EDTA, is mixed with 0125 ml 2X HBS (280 mM NaCl, 1.5 mM NaHPO$_4$, 40 mM HEPES pH 7.1). Then 33 ml 2M $CO_2$ is added and the precipitate allowed to form for 30 minutes at room temperature. The precipitate is then added to a 25 cm2 monolayer of Hela cells, along with 4 ml Dulbeco's modified Eagle's medium plus 10% fetal calf serum (DME). 12 hours later the precipitate is removed and 5 ml fresh DME is added to the cells. 24 hours later the transfected cells are split 1:20 into DME + 200 $\mu$g/ml G418. Surviving cells have incorporated the plasmid DNA and express human renin.

## Claims

1. Genomic DNA fragment coding for human renin which has the sequence shown in Figures 1A and 1B or a DNA fragment which codes for the same amino acid sequence and which contains the introns as indicated in Figures 1A and 1B.

2. An animal cell transformed by DNA as defined in claim 1.

## Revendications

1. Fragment d'ADN génomique codant pour la rénine humaine, qui comprend la séquence représentée sur les figures 1A et 1B, ou bien fragment d'ADN qui code pour la même séquence d'amino-acides et qui contient les introns indiqués sur les figures 1A et 1B.

2. Cellule animale transformée par l'ADN suivant la revendication 1.

## Patentansprüche

1. Genomische DNS-Fragmentkodierung für menschliches Renin hat die in Figuren 1A und 1B angegebene Sequenz oder ein DNS-Fragment, das für dieselbe Aminosäurensequenz kodiert und das die wie in Figuren 1A und 1B angegebenen Introne enthält.

2. Tierzelle, die durch die in Anspruch 1 definierte DNS umgeformt wird.

FIGURE 1A

EP 0 161 866 B1

-548   -500   -450
ACGTGTGAGCCACCATGCCTGGCCCCTCTACTCTTATAATTAAACCAGCTGTTGCTTTTCCTGCCAAGAAACCAGTCATGAAGATTCACCCATGTTCTAGATGGGAAAAC

-400   -350   oooooooooo
TGGGCTGTAGCCTGGGAGAGGCCAGTCAGGGACAAAGCCAAAGTTAATATAGAGAATGGAGCTTCCAGGGTATAGGGGTTGGGTCTGGGCTAGGGAGCTGGAAACCTAGG

ooo   -300   -250
TTTTACGCTTGTCCCAGTTTTGATGTTAGCCCTGAGCAGTGCTGTTTCTCATCAGCCTCTGCCTGCTCCAGGGGTCACAGGGCCAAGCCAGATAGAGGGCTGCTAGCGTC

-200   -150   ΔΔΔΔ
ACTGGACACAAGATTGCTTTCCCACAGCTGTCCTTCCTCCAGCCCCTCTGCTGGGGATCCGGAAACCTGGGTACCCTTCACCCACCTAGCTCTGTCCCGCAGTGAGATTT

ΔΔΔ   -100   ΔΔΔΔΔΔΔ   -50   xxxxxxxxxx   -1+1
ATTGCTGACTGCCCTGCCATCTACCCCAGGGTAATAAATCAGGGCAGAGCAGAATTGCAATCACCCCATGCATGGAGTGTATAAAAGGGGAAGGGCTAAGGGAGCCACAG

MetAspGly                    PheLysAr
AACCTCAGTGGATCTCAGAGAGAGCCCCAGACTGAGGGAAGCATGGATGGA-Exon_1(98_bp)-TTTAAACG GTAATTGGTAA---------CCTCCACCCTGGGCC

gIlePheLeu              TyrMetAsp                                                      ThrG
AG GATCTTCCTC-Exon_2(151_bp)-TACATGGAC GTGAGTGCTTGGCTCAGCCCCTCGCTCCCTCCCTG---------GCCCTCTCTTTTTACCCCCACAG ACCC

InTyr ... ThrAlaCysV
AGTAC-_Exon_3(124_bp)_-ACTGCCTGTG | GTGAGACCTA--------GTCCCCCTGCCAG | alTyrHis_Exon_4(119_bp)_IIeIIeThr
TGTATCAC-_____-ATCATCACC | GTAAGTTGGGCC

ValGlyGly ... TyrAsnAr
G--------TCCTCTGGTCCTTCCTCCCACAG | GTGGGTGGA-Exon_5(197_bp)_-TACAACAG | GTGGCGACTGGGACTCCAAGGGCTGAGGTGGGGGGACAGGAGG

Exon 6
gAspSerGl
GGAGAAGAGATGGGGAGTGGAAGGAGAG--------CCTTTCTGCTGTCTCTCTTTCTCTGCAG | AGATTCCGA | GTAAGGAGACAAAACCCCCACATGGCTGTGACCTT

uAsnSerGln_Exon_7(120_bp)_MetLysGl
CCAG--------CTTGCTGGGTCCTGGAGGTTATGGGTTTCCAAGAGCTTCTGATCTTTCCTTTAG | GAATTCCCAA-_____-ATGAAGGG | GTCAGAAA

yValSerVal_Exon_8(142_bp)_LeuPheAsp
TCCTCAACCCTCCCCGGGCTCC--------GGCCTCCCCCAG | GGTGTCTGTG-_____-CTGTTTGAT | GTAAGAAGCCAAAGAGGG--------ACTCA

TyrValVal_Exon_9(99_bp)_ValPheGln
AGAAGGCTCTCTTTGCCCCCCACCCCAG | TATGTCGTG-_____-GTATTTCAG | GTGAGGTTCGAGTCGGCCCCCTCGGTGG--------CGGGGTCAGGC

GluSerTyr_Exon_10(159_bp)_LeuAlaArg
AAGGTGAAAGGAGAATGCTCATGTGCTGGGTATGGAGAAACTCTCCCCCTTCCTGCCAG | GAATCCTAC-_____-TTGGCCCGCTGAGGCCCTCTGCCA

CCCAGGCAGGCCCTGCCTTCAGCCCTGGCCCAGAGCTGGAACACTCTCTGAGATGCCCCTCTGCCTGGGCTTATGCCCTCAGATGGAGACATTGGATGTGGAGCTCCTGC

TGGATGCGTGCCCTGACCCCTGCACCAGCCCTTCCCTGCTTTGAGGACAAAGAGAATAAAGACTTCATGTTCAC | AGCCTGTTGCATCTGGGTT

FIGURE 1B

EP 0 161 866 B1

# FIGURE 2